# EUROPÄISCHE PATENTSCHRIFT

(11) **EP 1 221 959 B1**
(45) Veröffentlichungstag und Bekanntmachung des Hinweises auf die Patenterteilung: **07.09.2005**
(21) Anmeldenummer: 00987046.0
(22) Anmeldetag: 26.10.2000
(51) Int. Cl.: A61K 35/76, A61P 35/00, A61P 37/00

(54) **VERWENDUNG VON PARVOVIREN ZUR VERBESSERUNG DES ALLGEMEINZUSTANDES BEI TUMORPATIENTEN ODER PATIENTEN MIT CHRONISCHEN ODER KONSUMIERENDEN ERKRANKUNGEN**
USE OF PARVOVIRUSES FOR IMPROVING THE GENERAL CONDITION OF TUMOUR PATIENTS OR PATIENTS HAVING CHRONIC OR CONSUMPTIVE DISEASES
UTILISATION DE PARVOVIRUS POUR AMELIORER L'ETAT GENERAL DE PATIENTS SOUFFRANT DE TUMEURS OU DE PATIENTS SOUFFRANT DE MALADIES CHRONIQUES OU DU TYPE CACHEXIQUE

(30) Priorität: 27.10.1999 DE 19951795
(43) Veröffentlichungstag der Anmeldung: 17.07.2002
(73) Patentinhaber: Von Knebel Doeberitz, Magnus, 69118 Heidelberg (DE)
(72) Erfinder: VON KNEBEL DOEBERITZ, Magnus, 69118 Heidelberg (DE); ZUR HAUSEN, Harald, 69483 Waldmichelbach (DE); SCHLEHOFER, Jörg, 69181 Leimen (DE); KLEIN-BAUERNSCHMITT, Petra, 69118 Heidelberg (DE); EISOLD, Sven, 69120 Heidelberg (DE)
(74) Vertreter: Schüssler, Andrea
(86) Internationale Anmeldenummer: PCT/DE2000/003827
(87) Internationale Veröffentlichungsnummer: WO 2001/030366

(56) Entgegenhaltungen:
- WO-A-97/12623
- WO-A-99/18799
- DE-A- 19 825 620
- KLEIN-BAUERNSCHMITT P ET AL: "IMPROVED EFFICACY OF CHEMOTHERAPY BY PARVOVIRUS-MEDIATED SENSITISATION OF HUMAN TUMOUR CELLS" EUROPEAN JOURNAL OF CANCER,GB,PERGAMON PRESS, OXFORD, Bd. 32A, Nr. 10, September 1996 (1996-09), Seiten 1774-1780, XP000867207 ISSN: 0959-8049
- HILLGENBERG M ET AL: "ENHANCED SENSITIVITY OF SMALL CELL LUNG CANCER CELL LINES TO CISPLATIN AND ETOPOSIDE AFTER INFECTION WITH ADENO-ASSOCIATED VIRUS TYPE 2" EUROPEAN JOURNAL OF CANCER,GB,PERGAMON PRESS, OXFORD, Bd. 35, Nr. 1, Januar 1999 (1999-01), Seiten 106-110, XP000867208 ISSN: 0959-8049
- EISOLD SVEN CHRISTIAN ET AL: "Enhanced sensitivity of pancreatic tumour cells to 5-FU-chemotherapy mediated by adeno-associated virus type 2 (AAV-2) infection in vitro and in vivo." GASTROENTEROLOGY, Bd. 118, Nr. 4 Suppl. 2 Part 1, April 2000 (2000-04), Seite AGA A531 XP000992794 101st Annual Meeting of the American Gastroenterological Association and the Digestive Disease Week.;San Diego, California, USA; May 21-24, 2000 ISSN: 0016-5085

## Beschreibung

Die Erfindung betrifft die Verwendung von Parvoviren zur Verbesserung des Allgemeinzustandes bei Tumorpatienten oder Patienten mit chronischen oder konsumierenden Erkrankungen.

Tumorpatienten, aber auch Patienten mit chronischen oder konsumierenden Erkrankungen, haben neben ihrer Erkrankungen auch oft unter einem schlechten Allgemeinzustand zu leiden. Dieser schlechte Allgemeinzustand äußert sich unter anderem in abnormen Blutwerten, Abgeschlagenheit, Mattigkeit und Appetitlosigkeit. Verstärkt wird dieser schlechte Allgemeinzustand oft noch durch eine notwendige Therapie mit genotoxischen Agentien. Dies gilt insbesondere für Patienten mit malignen Erkrankungen. Die Therapie maligner Erkrankungen basiert neben der chirurgischen Entfernung bisher vor allem auf der Anwendung genotoxischer Agentien, die als Chemo- oder Radiotherapeutika Tumorzellen angreifen und zerstören. In der Regel wird der Tod der Tumorzellen durch die Auslösung des programmierten Zelltods (Apoptose) hervorgerufen. Der Einsatz der genotoxischen Agentien wird jedoch durch das Auftreten von Nebenwirkungen, wie z.N. Nausea, Hyperemesis, Depression der Blutbildung, gastrointestinale Beschwerden, Blutungen usw., limitiert. Die genotoxischen Agentien können daher nicht in höherer Dosierung eingesetzt werden, als die maximal tolerierbaren Nebenwirkungen es erlauben. In vielen Fällen sind die Nebenwirkungen für den Patienten so ungenehm, ja sogar lebensgefährlich, daß die noch tolerierbaren Dosen des genotoxischen Stoffs nicht ausreichen, um alle Tumorzellen zu eliminieren. Dasselbe gilt für chronisch entzündliche Erkrankungen, jede Form der Autoimmunerkrankungen oder andere Indikationen, bei denen genotoxische Therapien, z.B. in Form von Chemo-, Radio- oder Immuntherapeutika eingesetzt werden. Es ist daher besonders wünschenswert, wenn Möglichkeiten bereitgestellt würden, die die Nebenwirkungen bei der Therapie abmildern, um so die Wirksamkeit der genotoxischen Agentien effizienter zu machen. Solche Ansätze sollten auch den allgemeinen klinischen Zustand von Tumorpatienten bzw. Patienten mit chronischen oder konsumierenden Erkrankungen verbessern.

Klein- Brenmschmitt et al., European Journal of Cancer Vol. 32A, No.10, pp. 1774-1780, 1996, beschriebt die Verwendung adeno-assozierter Viren zur verbesserten Wirksamkeit von Chemotherapie.

Der vorliegenden Erfindung liegt daher die Aufgabe zugrunde, den Allgemeinzustand von Tumorpatienten oder Patienten mit chronischen oder konsumierenden Erkrankungen deutlich zu verbessern. Weiter soll das Problem der durch genotoxische Therapie induzierten Nebenwirkungen vermindert werden. Dadurch sollen die genotoxischen Agentien in höherer Menge einsetzbar werden und vom Patienten auch in höherer Dosierung besser toleriert werden können, was die Effizienz der Behandlung steigert und die Überlebensrate von Patienten - verbessert.

Diese Aufgabe wird durch die Gegenstände der Patentansprüche gelöst.

Erfindungsgemäß werden durch die Verwendung von Parvoviren, insbesondere adenoassoziierten Viren die Nebenwirkungen gemildert, die ansonsten bei Patienten auftreten, die sich einer genotoxischen Therapie unterziehen müssen. Ferner wird der Allgemeinzustand der erkrankten Individuen deutlich verbessert. Dies gilt insbesondere für Tumorpatienten, weil die Ausbildung von Tumorkachexie und die durch eine neoplastische Erkrankung hervorgerufene Knochenmarksdepression deutlich reduziert wird.

Der vorliegenden Erfindung liegt die Beobachtung zugrunde, daß die Verabreichung von Parvoviren im Rahmen einer genotoxischen Therapie zu einer deutlichen Verminderung der dabei sonst üblichen Nebenwirkungen führt. Dies geht einher mit einer gesteigerten Wirkung (insbesondere anti-neoplastischen Wirkung) der Therapie, da jetzt auch höhere Dosen des genotoxischen Agens toleriert werden und die Dauer der Therapie ausgeweitet werden kann. So kann durch die kombinierte Anwendung von genotoxischer Therapie mit der Gabe von Parvoviren die Effektivität einer (Tumor)behandlung gesteigert werden, während gleichzeitig die Nebenwirkungen der genotoxischen Behandlung drastisch abgesenkt werden können. Dieser Effekt wird auch durch die wiederholte Applikation der gemeinsamen Gabe von genotoxischem Agenz und Parvoviren-Infektion nicht abgemindert. Durch diesen Ansatz kann die Wirksamkeit und vor allem die Verträglichkeit einer Chemo- oder Radiotherapie erheblich gesteigert werden.

Eine Infektion mit Parvoviren in Kombination mit genotoxischer Therapie, z.B. chemo- oder radiotherapeutischen Maßnahmen, steigert nach Erkenntnis der Erfinder die Effizienz der herkömmlichen Therapie und vermindert auftretende Nebenwirkungen, so daß eine weitere Therapie erfolgversprechender als bisher durchgeführt werden kann. Erfindungsgemäß können beliebige apathogene Parvoviren verwendet werden. Besonders erfolgsversprechend und somit bevorzugt sind adenoassoziierte Viren (AAV). Es können auch Fragmente von Parvoviren, insbesondere von AAV, verwendet werden, solange diese die gewünschte Wirkung ausüben können. Besonders bevorzugt wird das AAV-2-Virus oder Fragmente davon verwendet.

Das AAV-Virus ist ein humanes Parvovirus, das eine Koinfektion mit einem Helfervirus benötigt, damit eine produktive Infektion stattfinden kann. AAV infiziert Menschen in früher Kindheit und gilt als apathogen, da keine menschliche Krankheit mit der AAV-Infektion verbunden werden konnte (Adv. in Vir. Res. 1987, 32, 43-306). Ferner führt die AAV-Infektion auch nicht zur Ausbildung einer neutralisierenden Immunantwort, so daß Reinfektionen jederzeit vorkommen können. Seroepidemiologische Daten zeigen, daß die Inzidenz von Tumorerkrankungen bei Patienten mit Antikörpern gegen AAV seltener vorkommen als bei Patienten, die keine Antikörper gegen AAV aufwiesen.

In Tierversuchen mit immunkompetenten Lewis-Ratten, denen subkutan Pankreaskarzinomzellen implantiert wurden, konnte gezeigt werden, daß eine AAV-Infektion, die gleichzeitig mit einer chemotherapeutischen Behandlung vorgenommen wurde, zu einer verminderten Tumorprogression im Vergleich zu der Rattengruppe, die nur Chemotherapie erhalten hatte, führte. Außerdem wiesen die Ratten, die unter der Chemotherapie gleichzeitig mit AAV-2 infiziert worden waren, einen deutlich geringeren Gewichtsverlust auf, als die Tiere, die nur Chemotherapie erhalten hatten. Die Vigilanz der Tiere konnte ebenfalls durch kombinierte Gabe von AAV-2 und Chemotherapie deutlich verbessert werden. Auch die hämatologischen Parameter wurden durch die Chemotherapie in den AAV-2 infizierten Tieren deutlich weniger beeinträchtigt, als in den Tieren, die nur Chemotherapie erhalten hatten.

Die erfindungsgemäße Verwendung der Parvoviren, insbesondere AAV-Viren, kann ohne eine Zusatzbehandlung bzw. vor, gleichzeitig oder nach einer genotoxischen Behandlung erfolgen. Sie erfolgt jedoch bevorzugt vor der ersten genotoxischen Behandlung und wird mit jedem Behandlungszyklus, in dem das genotoxische Agenz verabreicht wird, wiederholt. Auch bei Tumoren, die durch die sonst üblicherweise verwendbaren Dosen an genotoxischem Agenz nicht therapierbar waren, kann so zumindest eine partielle Remission erzielt werden. Gleichzeitig führt die Parvoviren-Infektion zu einer erheblichen Verbesserung der gesamten physiologischen Situation einschließlich des Blutbildes und Erhalt des Körpergewichts.

Die genotoxische Therapie kann vorzugsweise eine beliebige Radio- oder Chemotherapie sein, die dem zu behandelnden Krebs angepaßt ist. Genotoxische Agentien im Rahmen dieser Therapien sind die dem Fachmann bestens bekannten Chemotherapeutika (s. unten) und verschiedenen Bestrahlungsquellen, z.B. α, β-, γ-Strahlen, Neutronen-, Protonen- oder anderen Strahlungsarten. Solche Therapien sind seit Jahren bekannt und neben der chirurgischen Entfernung des Tumors die etablierte Methode, um Krebserkrankungen zu heilen bzw. die Lebenserwartung eines Patienten um einige Zeit zu erhöhen. Dem Fachmann sind deshalb die Maßnahmen einer Radio- oder Chemotherapie bestens bekannt. Neben der klassischen bzw. Chemo- oder Radiotherapie schließt die Erfindung aber auch die Kombination von Parvovirus-Infektionen mit jeder anderen Form der anti-neoplastischen Therapie ein. Insbesondere die Kombination mit gentherapeutischen Ansätzen ist hier zu nennen. Erfindungsgemäß wird auch die Tumortherapie in jeglicher Form der Gen- oder Immuntherapie durch rekombinante Viren aber auch allen nicht-viralen Vektoren und Applikationen durch die gleichzeitige Gabe von Parvoviren unterstützt. Hierzu zählt insbesondere die Übertragung von sog. Tumorsuppressorgenen oder sog. Suizidgenen, der Einsatz von Antikörpern, Zytokinen und anderen Medikamenten jeglicher Art oder die gegen Tumorzellen gerichtet sind. Dies umfaßt auch die Therapie mit allen immunmodulatorischen Ansätzen einschließlich allen Vekzinierungsmaßnahmen, um gegen die Erkrankung eine Immunantwort aufzubauen.

Die erfindungsgemäße Verwendung der Parvoviren, insbesondere AAV-Viren, kann auf beliebige Krebsarten angewendet werden, wobei beste Erfolge bei allen gastrointestinalen Tumoren sowie Tumoren des Respirations- und oberen Verdauungstrakts als auch der ableitenden Hamwege, des Nervensystems, der Geschlechtsorgane sowie aller mesenchymaler Gewebe und des blutbildenden Systems zu erwarten sind. Bevorzugt sind Colon-, Lungen-, Pankreas- und Himtumoren (insbesondere Glioblastom) zu nennen. Die Erfindung schließt aber auch alle anderen Indikationen, bei denen Chemo- oder Radiotherapeutika eingesetzt werden, mit ein. Dies betrifft insbesondere chronisch entzündliche Erkrankungen (z.B. rheumatoide Arthritis und weitere Erkrankungen des rheumatischen Formenkreises), jede Form der Autoimmunerkrankungen (z.B. Morbus Crohn, - Colitis ulcerosa, usw.) oder andere Indikationen, bei denen Chemo-, Radio- oder Immuntherapeutika eingesetzt werden. Ebenfalls eingeschlossen sind hier alle chronisch konsumierenden Erkrankungen (z.B. Tumorerkrankungen jeder Art, HIV-Infektionen usw.) bei denen der Allgemeinzustand der Patienten durch Gabe von Parvoviren verbessert werden kann.

Es zeigte sich ferner, daß schon die alleinige Gabe von Parvoviren, insbesondere AAV, ohne Chemotherapie den Zustand von Tieren mit chronisch konsumierenden Erkrankungen, wie z.B. einem wachsenden Tumor, deutlich verbessert werden kann. Daher schließt die Erfindung auch jegliche Form der Verabreichung von Parvoviren bei chronischen, degenerativen und/oder konsumierenden Krankheitszuständen mit dem Ziel der Verbesserung des Allgemeinzustandes ein.

Die erfindungsgemäße Verwendung erfolgt intravenös, subkutan, intraarteriell, intraperitoneal, intramuskulär, intratumoral, peroral (auch per inhalationem) oder kutan. Ferner kann das Parvovirus durch spezielle Drogenapplikationssysteme unter pharmakologischen Aspekten direkt am Ort der Erkrankung freigesetzt werden. Dabei wird das Virus in einem geeigneten, dem Verabreichungsweg angepaßten Präparat formuliert. So ist es bevorzugt für eine intravenöse (auch als Infusion) und intratumorale Verabreichung das Virus in einer physiologischen Kochsalzlösung, Ringerlösung oder PBS-Lösung (Phosphat-gepufferte Salzlösung), für eine kutane Verabreichung in Form einer Salbe, Suspension oder Gel, für eine orale Verabreichung in Form von Tabletten oder Dragees, sowie für Inhaltionszwecke als Aerosolspray bereitzustellen.

Die verwendete Virusdosis beträgt abhängig vom Körpergewicht des Patienten 10⁹-10¹⁰ Virus-Partikel/kg KG. Die Dosis wird jedoch von einem Arzt festgesetzt und ist abhängig von Gewicht, Alter und Geschlecht des Patienten sowie Schwere der Erkrankung, Art der Verabreichung und Dauer der Verabreichung. Eine erfindungsgemäße Zusammensetzung kann auch übliche Hilfsstoffe enthalten. Als Hilfsstoffe können die üblichen, wie Arzneimittelträger, Bindemittel, Sprengmittel, Gleitmittel, Lösungsmittel, Lösungsvermittler, Freigabe-Beschleuniger, Freigabe-Verzögerer, Emulgatoren, Stabilisatoren, Färbemittel oder Geschmackskorrgenzien verwendet werden.

Erfindungsgemäß wird auch eine pharmazeutische Zusammensetzung bereitgestellt, die neben einem genotoxischen Agenz, insbesondere einem Chemotherapeutikum (Zytostatikum), Parvoviren, insbesondere adeno-assoziierte Viren, z.B.AAV-2, enthält. Als Chemotherapeutikum sind alle bisher in der Tumortherapie oder anderen Therapien oben genannter Erkrankungen gebräuchlichen Chemotherapeutika (Zytostatika) einzeln oder in Kombination einsetzbar, beispielsweise Cis-Platin und seine Derivate, Etoposid, Methothrexat, Doxorubicin, Cyclophosphamid, Ifosphamid, Trofosfamid, Busulfan, Cytarabin, Fluoruracil, Mercaptopurin, Vinblastinsulfat, Vincristinsulfat, Bleomycinsulfat, Taxol oder Mitomycin.

Die Erfindung wird anhand des nachfolgenden Beispiels näher erläutert.

### Beispiel 1

In Tierversuchen mit immunkompetenten Lewis-Ratten wurden syngene DSL6A-Pankreaskarzinomzellen mit einer Tumorzellzahl von 1x10⁶ subcutan an Tieren in der Bauchleiste inokuliert. Diese Zellen wuchsen in allen so behandelten Tieren zu Tumoren an. Nach 4 bzw. 6 Wochen erreichten die Tumoren eine durchschnittliche Größe von 30 mm² respektive 60 mm². Es wurden zwei unterschiedliche Dosierungen für 5-Fluoruracil (5-FU), (5 mg/kg/KG (Minimaldosis) bzw. 50 mg/kg/KG (Maximaldosis) einmal wöchentlich pro Tier verwendet. Tiere mit einer Tumorgröße von durchschnittlich von 30 mm² vier Wochen nach Tumorinokkulation wurden mit der Minimaldosierung für 5-FU (5 mg/kg/KG) behandelt. Demgegenüber wurden Tiere mit einer Tumorgröße von durchschnittlich 60 mm² sechs Wochen nach Tumorinokkulation mit der Hochdosis für 5-FU (50 mg/kg/KG) therapiert. Zur Untersuchung des Effekts einer gleichzeitigen AAV-2-Infektion erfolgte die wöchtenliche Applikation von AAV-2 mit 1x10⁸ Viruspartikel intratumoral pro Versuchstier. Als Kontrollgruppe dienten tumortragende Tiere ohne Behandlung. Um den Effekt einer alleinigen AAV-Infektion auf das Tumorwachstum und die physiologischen Veränderungen in den Versuchstieren kontrollieren zu können, wurden tumortragenden Lewis-Ratten mit 1x10⁸ AAV-2-Viruspartikel intratumoral infiziert (Tabelle 1)

| Anzahl der Tiere | Beschreibung |
|---|---|
| 12 | Kontrolle, ohne Therapie |
| 9 | 5-FU, Dosis 5 mg/kg/KG (1x wö., i.p.) |
| 13 | 5-FU, Dosis 5 mg/kg/KG (1x wö., i.p.) + AAV-2 (1x10⁸ Viruspartikel wö., i.t.) |
| 12 | 5-FU, Dosis 50 mg/kg/KG (1x wö., i.p.) |
| 12 | 5-FU, Dosis 50 mg/kg/KG (1 x wö., i.p.) + AAV-2 (1x10⁸ Viruspartikel wö., i.t.) |
| 12 | AAV-2 ((1x10⁸ Viruspartikel wö., i.t.) |

Für alle Tiere wurde wöchentlich die Tumorgröße (Länge x Breite), das Auftreten von Metastasen und das Gewicht bestimmt. Nach den Empfehlungen der Arbeitsgruppe der LASA (Laboratory Animal Science Association) wurde ein SCORE entwickelt und für die Versuchstiere einmal wöchtenlich evaluiert (Report of the laboratory animal science association working party, in Laboratory animals, 1990, (24), 97-130).

| | **3 Punkte** | **2 Punkte** | **1 Punkt** |
|---|---|---|---|
| **Vigilanz/Verhalten** | normal | träge oder unruhig | schläfrig, bewußtlos |
| **Haltung/Motorik** | normal | partiell eingeschränkt | gelähmt, immobil |
| **Haarkleid** | normal | matt, gesträubt | Haarausfall |
| **Ernährungszustand** | normal | reduziert | abgemagert, dehydriert |
| **Schmerz** | klein | druckempfindlich | Schonhaltung, Laute |

Nach 5-6 Behandlungswochen wurde für jeweils 6 Tiere der verschiedenen Versuchsgruppen 1 ml Blut aus der Schwanzvene entnommen und die Hämatologischen Parameter; Leukozytenzahl (gesamt), Hämoglobin, Hämatokrit, Thrombozytenzahl und das Differentialblutbild (Neutrophile, Lymphozyten, Monozyten) untersucht.

Bei einer Gewichtsabnahme von > 20% erfolgte eine Unterbrechung der Behandlung. Endpunkt der Untersuchung war eine expansive Tumorgröße von > 400 mm² bzw. Auftreten von mehr als 3 Metastasen.

### ERGEBNISSE

Im Verlauf dieses Experimentes stellte sich heraus, daß die Tiere, die den Tumor trugen und nicht behandelt wurden (Kontrollgruppe), ein rasches Tumorwachstum aufwiesen. In der Folge kam es zu Gewichtsverlust, einer Leukopenie im Blutbild und einer prozentual verstärkten Abnahme der polymorphkemigen Granulozyten und Monozyten im Differentialblutbild im Vergleich zu gesunden Tieren ohne Tumor. Die Tiere, die mit AAV-2 infiziert wurden, hatten trotz progredientem Tumorwachstum eine altersentsprechende Gewichtszunahme und zeigten keine Leukopenie. Insgesamt waren diese Tiere sehr viel vitaler und wiesen einen signifikant besseren LASA-Score zur Beurteilung des Allgemeinzustandes auf.

**Tabelle:**

| Vergleich tumortragende Tiere ohne Therapie und tumortragende Tiere mit AAV-Infektion nach maximal 10 Wochen post Tumorinokkulation (durchschnittliche Angabe) | | | | |
|---|---|---|---|---|
| | Gewicht (g) | Leukozyten (pro µl) | LASA-Score | Überlebensrate |
| Tumortragende Tiere ohne Therapie | 321 | 3067 | 3 | 0/12 |
| Tumortragende Tiere mit AAV-Infektion | 372 | 8700 | 13 | 8/12 |

Bei einer durchschnittlichen Tumorgröße von 30 mm² vier Wochen nach Tumorinokkulation zeigte die alleinige Chemotherapie mit 5-FU in einer Dosierung von 5 mg/kg/KG keinen inhibitorischen Effekt auf das Tumorwachstum. Demgegenüber konnte die Kombinationsbehandlung mit 5-FU der gleichen Dosierung (5 mg/kg/KG) und einer gleichzeitigen AAV-Infektion einen Wachstumsstop des Tumors für 2-3 Wochen bewirken. Danach zeigte sich eine verminderte Tumorprogression im Vergleich zu den ausschließlich 5-FU (5 mg/kg/KG) therapierten Tieren. So waren von 13 Tieren nach 6 Behandlungwochen alle Tiere am Leben, während bei den allein chemotherapierten Tieren 5 von 9 Tieren verstorben waren. Darüberhinaus wiesen die zusätzlich mit AAV-infizierten Tiere einen besseren LASA-Score und eine größere Anzahl von Leukozyten im Vergleich zu den ausschließlich chemotherapierten Tieren auf.

**Tabelle:**

| Vergleich 5-FU-Monotherapie (5 mg/kg/KG, Minimaldosis) und Kombinationsbehandlung von 5-FU (5 mg/kg/KG) mit gleichzeitiger AAV-Infektion nach 6 Behandlungswochen (durchschnittliche Angabe). | | | | |
|---|---|---|---|---|
| | Gewicht (g) | Leukozyten (pro µl) | LASA-Score | Überlebensrate |
| 5-FU (5mg/kg/KG) | 355 | 6843 | 9 | 4/9 |
| 5-FU (5mg/kg/KG + AAV-2 | 354 | 9500 | 15 | 13/13 |
| * Behandlungsbeginn vier Wochen nach Tumorinokkulation (TU-Größe ∼ 30 mm²) | | | | |

Sechs Wochen nach Tumorinokkulation erreichten die Tumore eine durchschnittliche Größe von 60 mm². Für diese Tiere erfolgte die 5-FU-Chemotherapie mit der Maximaldosis von 50 mg/kg/KG entweder als Monotherapie oder in Kombination mit der AAV-Infektion. Dabei konnte für die Tiere die ausschließlich mit 5-FU behandelt wurden, ein kurzfristiger Stillstand des Tumorwachstums erreicht werden. Im weiteren Therapieverlauf zeigten sich erhebliche Nebeneffekte der Chemotherapie. So mußte nach der zweiten Behandlungswoche aufgrund des schlechten Allgemeinzustandes der Tiere und einer durchschnittlichen Gewichtsabnahme von mehr als 20% die Therapie ausgesetzt werden. Tiere mit einer zusätzlichen AAV-Infektion hatten diese Nebenwirkungen nicht und wiesen initial eine Abnahme des Tumors auf. Anschließend sistierte das Tumorwachstum für ca. 4 Wochen und die Überlebenszeit der Ratten wurde gegenüber den kontrollierten bzw. den mit ausschließlich 5-FU behandelten Tieren signifikant verlängert. So waren nach 6 Behandlungswochen von den ausschließlich Hochdosis chemotherapierten Tieren 9 von 12 Ratten verstorben. Demgegenüber waren in der Gruppe mit den zusätzlich AAV-infizierten Tieren nur 3 von insgesamt 12 Ratten verstorben. Die Bestimmung der Verlaufsparameter 5-6 Wochen nach Behandlungsbeginn demonstrierte für chemotherapierte Tiere ohne gleichzeitige Infektion mit AAV-2 eine ausgeprägte Leukopenie, einen erniedrigten LASA-Score und ein deutlich reduziertes Gewicht. Bei den Tieren, die sowohl die Chemotherapie als auch die AAV-Infektion erhielten, konnte keine Chemotherapie- bzw. Tumorinduzierte Leukopenie festgestellt werden. Diese Tiere waren erheblich vitaler (LASA-Score) und wiesen keinen Gewichtsverlust auf.

**Tabelle:**

| Vergleich 5-FU-Monotherapie (50 mg/kg/KG, Hochdosis) und Kombinationsbehandlung von 5-FU (50 mg/kg/KG) mit gleichzeitiger AAV-Infektion nach 6 Behandlungswochen (durchschnittliche Angabe). | | | | |
|---|---|---|---|---|
| | Gewicht (g) | Leukozyten (pro µl) | LASA-Score | Überlebensrate |
| 5-FU (50mg/kg/KG) | 306 | 640 | 6 | 3/12 |
| 5-FU (50mg/kg/KG) + AAV-2 | 353 | 8325 | 14 | 9/12 |
| *Behandlungsbeginn sechs Wochen nach Tumorinokkulation (TU-Größe ∼ 60 mm²) | | | | |

Der signifikant bessere Allgemeinzustand der zusätzlich mit AAV-infizierten Tiere und die dadurch verminderten Nebenwirkungen der Hochdosis-Chemotherapie (5-FU, 50 mg/kg/KG) spiegelte sich in allen untersuchten hämatologischen Parametern wieder. So hatten die ausschließlich mit 5-FU (50 mg/kg/KG) behandelten Tiere signifikant erniedrigte Werte für das Hämoglobin, die Thrombozytenzahl und die im Differentialblutbild bestimmten Werte für Neutrophile, Lymphozyten und Granulozyten.

**Tabelle:**

| Vergleich der hämatologischen Parameter von 5-FU-Monotherapie (50 mg/kg/KG, Hochdosis) und von 5-FU (50 mg/kg/KG) mit gleichzeitiger AAV-Infektion nach 6 Behandlungswochen (durchschnittliche Angabe). | | | | | |
|---|---|---|---|---|---|
| | Hämoglobin (g/dl) | Thrombozyten (pro µl) | Neutrophile (pro µl) | Lymphozyten (pro µl) | Monozyten (pro µl) |
| 5-FU (50mg/kg/KG) | 6,5 | 76.000 | 18 | 606 | 6 |
| 5-FU (50mg/kg/KG) + AAV-2 | 11,6 | 540.000 | 353 | 6503 | 1002 |
| Normwerte Ratte | 13,5 | 621.000 | 820 | 9100 | 220 |
| * Behandlungsbeginn sechs Wochen nach Tumorinokkulation (TU-Größe ~ 60 mm²) | | | | | |

Diese Arbeiten zeigen, daß die begleitende Infektion von tumortragenden Ratten mit adeno-assoziierten Viren (z.B. AAV-2) zu einer erheblichen Verbesserung des Blutbildes und des gesamten physiologischen Zustandes sowie zu einer Verminderung des Gewichtsverlustes der Tiere führt.

## Patentansprüche

1. Verwendung von apathogenen Parvoviren zur Herstellung eines Arzneimittels zur Verminderung der Nebenwirkungen von Therapien mit genotoxischen Agentien bei Patienten mit chronischen und/oder konsumierenden Erkrankungen, wobei die Verminderung der Nebenwirkungen bei Aufrechterhaltung oder Steigerung der Dosis der genotoxischen Agentien auftritt und die Nebenwirkungen hämatologische Parameter betreffen.

2. Verwendung nach Anspruch 1, wobei die verwendeten Parvoviren adeno-assoziierte Viren sind.

3. Verwendung nach Anspruch 2, wobei die adeno-assoziierten Viren AAV-2 sind.

4. Verwendung nach einem der Ansprüche 1 bis 3, wobei die Parvoviren in einer Dosis von 10⁹-10¹⁰ Partikel/kg KG einzusetzen sind.

5. Verwendung nach einem der Ansprüche 1 bis 4, wobei die Erkrankung eine Tumorerkrankung ist.

6. Verwendung nach einem der Ansprüche 1 bis 5, wobei die Gabe der Parvoviren intravenös, subkutan, oral, intraperitoneal, intraarteriell oder intratumoral zu erfolgen hat.

7. Verwendung nach einem der Ansprüche 1 bis 6, wobei die Gabe der Parvoviren vor oder gleichzeitig zu der Therapie mit genotoxischen Agentien zu erfolgen hat.

8. Verwendung nach einem der Ansprüche 1 bis 7, wobei die genotoxischen Agentien im Rahmen einer Chemo- und/oder Radiotherapie, einer Gentherapie oder einer Immuntherapie zu geben sind.

## Claims

1. Use of non-pathogenic parvoviruses for the production of a medicament for reducing the side-effects of therapies with genotoxic agents in patients suffering from chronic and/or consumptive diseases, the reduction of the side-effects occurring while the dose of the genotoxic agents is maintained or raised and the side-effects relating to hematological parameters.

2. Use according to claim 1, wherein the parvoviruses used are adeno-associated viruses.

3. Use according to claim 2, wherein the adeno-associated viruses are AAV-2.

4. Use according to any of claims 1 to 3, wherein the parvoviruses are to be used in a dose of 10⁹-10¹⁰ particles/kg body weight.

5. Use according to any of claims 1 to 4, wherein the disease is a tumoral disease.

6. Use according to any of claims 1 to 5, wherein the parvoviruses are to be administered intravenously, subcutaneously, orally, intraperitoneally, intraarterially or intratumorally.

7. Use according to any of claims 1 to 6, wherein the parvoviruses are to be administered before, or simultaneously with, the therapy with genotoxic agents.

8. Use according to any of claims 1 to 7, wherein the genotoxic agents are to be administered based on a chemotherapy and/or radiotherapy, a gene therapy or an immunotherapy.

## Revendications

1. Utilisation de parvovirus non pathogènes pour la fabrication d'un médicament destiné à réduire les effets secondaires des thérapies utilisant des agents génotoxiques chez des patients atteints de maladies chroniques et/ou consomptives, la réduction des effets secondaires se manifestant lors du maintien ou de l'augmentation de la dose des agents génotoxiques et les effets secondaires concernant des paramètres hématologiques.

2. Utilisation selon la revendication 1, dans laquelle les parvovirus utilisés sont des virus adéno-associés.

3. Utilisation selon la revendication 2, dans laquelle les virus adéno-associés sont de type VAA-2.

4. Utilisation selon l'une des revendications 1 à 3, dans laquelle les parvovirus doivent être utilisés à une dose de 10⁹-10¹⁰ particules/kg PC.

5. Utilisation selon l'une des revendications 1 à 4, dans laquelle la maladie est une tumeur.

6. Utilisation selon l'une des revendications 1 à 5, dans laquelle les parvovirus doivent être administrés par voie intraveineuse, sous-cutanée, orale, intrapéritonéale, intra-artérielle ou intratumorale.

7. Utilisation selon l'une des revendications 1 à 6, dans laquelle les parvovirus doivent être administrés avant ou en concomitance avec la thérapie utilisant des agents génotoxiques.

8. Utilisation selon l'une des revendications 1 à 7, dans laquelle les agents génotoxiques doivent être administrés dans le cadre d'une chimiothérapie et/ou d'une radiothérapie, d'une thérapie génique ou d'une immunothérapie.
